# FASCICULE DE BREVET EUROPEEN

(11) **EP 3 194 295 B1**
(45) Date de publication et mention de la délivrance du brevet: **23.07.2025**
(21) Numéro de dépôt: 15778365.5
(22) Date de dépôt: 18.09.2015
(51) Int. Cl.: B32B 27/32, C07D 493/04

(54) **PROCÉDÉ DE CONDITIONNEMENT D'UN DIANYDROHEXITOL, SOLUTION AQUEUSE DE DIANHYDROHEXITOL CONDITIONNÉE ET SES UTILISATIONS**
VERFAHREN ZUR KONDITIONIERUNG EINER WÄSSRIGEN DIANHYDROHEXITOLLÖSUNG AUS AUFBEREITETEM DIANHYDROHEXITOL UND VERWENDUNGEN DAVON
METHOD FOR CONDITIONING A DIANHYDROHEXITOL, AQUEOUS SOLUTION OF CONDITIONED DIANHYDROHEXITOL, AND USES THEREOF

(30) Priorité: 19.09.2014 FR 1458868
(43) Date de publication de la demande: 26.07.2017
(73) Titulaire: Roquette Frères, 62136 Lestrem (FR)
(72) Inventeur: IBERT, Mathias, F-59930 La Chapelle d'Armentieres (FR); WYART, Hervé, F-62149 Cuinchy (FR); PARENT, Gwenaëlle, F-62350 Calonne sur la Lys (FR)
(74) Mandataire: Plasseraud IP
(86) Numéro de dépôt international: PCT/FR2015/052511
(87) Numéro de publication internationale: WO 2016/042277

(56) Documents cités:
- EP-B1- 1 287 000
- WO-A2-2009/019371
- FR-A1- 2 978 666
- MICHAEL ASH, IRENE ASH: "Handbook of Preservatives", 2004, SYNAPSE INFORMATION RESOURCES, INC., U.S.A., ISBN: 1-890595-66-7, article "Sodium phosphate", pages: 539, XP002739513
- LAWRENCE H. KEITH, MARY WALKER: "Handbook of Air Toxics: Sampling, Analysis, and Properties", 1 January 1995, CRC PRESS, INC., ISBN: 1-56670-114-7, pages: 312, XP002739521

## Description

La présente invention concerne un procédé de conditionnement de dianhydrohexitol sous forme de solution aqueuse dans un matériau d'emballage comprenant au moins une couche à base de polyoléfine.

Il s'agit d'un procédé extrêmement simple dans sa mise en œuvre et notamment beaucoup moins complexe que les méthodes de l'art antérieur essentiellement réservées à des produits solides, qui recommandent d'utiliser des matériaux d'emballage imperméables aux gaz, et de travailler sous pression réduite en oxygène et en azote.

Le procédé selon la présente invention s'avère également peu coûteux, ce qui constitue un autre avantage. En outre, la société demanderesse a démontré que seul un dianhydrohexitol sous forme de solution aqueuse -et non un produit solide- était apte à être conservé de manière stable dans ce type d'emballage. Comme ledit emballage contient peu, et préférentiellement ne contient pas d'antioxydant, on dispose ainsi d'un procédé permettant d'emballer un dianhydrohexitol pour des applications où l'on cherche à minimiser -voire à exclure- les antioxydants, comme les secteurs pharmaceutiques et alimentaires.

La présente invention concerne également la solution aqueuse de dianhydrohexitol ainsi emballée, et toutes ses utilisations dont notamment celles dans les domaines alimentaires et pharmaceutiques.

Les dianhydrohexitols, également appelés isohexides, sont des produits de déshydratation interne de sucres hydrogénés en C6 (hexitols) tels que le sorbitol, le mannitol et l'iditol. Parmi ces sucres hydrogénés doublement déshydratés, l'isosorbide est aujourd'hui celui pour lequel on envisage le plus d'applications industrielles, notamment dans le secteur des matières plastiques, comme intermédiaire de synthèse chimique, mais aussi dans les domaines alimentaires et pharmaceutiques.

Pour la majorité de ces applications, il est généralement nécessaire de disposer de compositions les plus pures possible, ayant notamment une teneur en dianhydrohexitols au moins égale à 98,5 % en poids, de préférence au moins égale à 99,5 % en poids par rapport à leur matière sèche totale. Or, les dianhydrohexitols et en particulier l'isosorbide sont des produits fortement hygroscopiques et chimiquement peu stables. La société demanderesse a en particulier observé que le stockage d'isosorbide fabriqué selon des procédés connus, même à l'abri de l'humidité de l'atmosphère, pouvait entraîner dans certaines conditions de température une dégradation chimique aboutissant, entre autres, à la formation d'acide formique, acide qui présente une odeur caractéristique et désagréable, particulièrement gênante dans des applications pharmaceutiques ou autres.

La société demanderesse a ainsi été amenée à mettre au point des procédés de purification et de stabilisation de dianhydrohexitols décrits notamment dans les demandes de brevets EP 1 287 000 et WO 03 / 043959. Les inventions relatives à ces demandes portent aussi bien sur des dianhydrohexitols solides que sur des dianhydrohexitols sous forme liquide. Une composition liquide stable d'isosorbide avec une teneur en matière sèche comprise entre 50 % et 90 % est divulguée dans le document WO 03 / 043959. Ceci étant, seule la stabilité des produits solides a été évaluée dans ces deux demandes, par stockage des échantillons à tester dans un récipient respectivement en plastique dans des saches de polyéthylène (EP 1 287 000) et en verre (WO 03 / 043959), a une température respectivement égale à 60°C et 40°C.

Par la suite, la société demanderesse s'est aperçue que les durées de conservation des dianhydrohexitols solides, déterminées dans les conditions des tests décrits dans les demandes de brevet EP 1 287 000 et WO 03 / 043959, ne reflétaient qu'imparfaitement la stabilité de ces mêmes produits dans les conditions réelles de transport et de stockage. La société demanderesse a relevé, en particulier dans certains cas, des concentrations relativement importantes en acide formique à proximité du film en polyéthylène servant à emballer classiquement des dianhydrohexitols sous forme solide, et notamment de l'isosorbide à l'état de poudre.

La société demanderesse a alors constaté qu'en augmentant de manière substantielle la concentration en agents antioxydants de la couche de matière plastique en contact avec le dianhydrohexitol, on améliorait sensiblement la stabilité de ce denier, notamment lorsque le dianhydrohexitol en question était de l'isosorbide. Cette invention a été protégée dans la demande de brevet WO 2009 / 019371, le matériau plastique d'emballage étant choisi parmi le polyéthylène, le polypropylène et les copolymères d'éthylène et de propylène.

Il est à noter que le procédé d'emballage objet de ladite demande s'applique à des dianhydrohexitols liquides ou solides même si la forme solide est privilégiée, et qu'une variante toute préférée consiste à emballer le dianhydrohexitol sous atmosphère anhydre et / ou inerte, par exemple sous atmosphère d'azote. Ce document stipule que pour garantir une stabilité optimale du dianhydrohexitol au cours du transport et du stockage, il est nécessaire de prévoir une couche supplémentaire de protection contre l'oxygène de l'air, la vapeur d'eau et / ou la lumière.

En outre, il convient de noter que ce type de solution ne permet pas de conditionner des dianhydrohexitols pour des applications alimentaires ou pharmaceutiques, qui toutes deux requièrent des quantités très réduites en agents antioxydants. A titre d'exemple, la FDA (Food and Drug Agency) promulgue une liste des seuls antioxydants et stabilisants autorisés dans ces deux applications, avec toutes les restrictions correspondantes en terme de quantités en fonction du matériau polymère dans lequel ils sont incorporés.

Indépendamment de ce contexte alimentaire ou pharmaceutique, il s'est avéré que les solutions techniques développées dans la précédente invention nécessitaient l'élaboration de matériaux de conditionnement complexes et ne s'appliquaient qu'à de faibles quantités (une dizaine de grammes) de dianhydrohexitols, le problème du compactage des dianhydrohexitols lors de leur stockage en sacs de plusieurs kilogrammes voire plusieurs dizaines de kilogrammes restant sans solution.

Ce dernier problème a été résolu dans la demande de brevet WO 2013 / 021126, à travers la mise au point d'un procédé d'emballage de dianhydrohexitol dans un matériau de conditionnement imperméable aux gaz, caractérisé en ce que la pression partielle de l'oxygène à l'intérieur du conditionnement est comprise entre 0,1 mbar et 10 mbar. Une variante préférée consiste à limiter également la pression partielle de l'azote à l'intérieur du conditionnement entre 240 et 1012,9 mbar. Il est bien mentionné que ces types de conditionnement conviennent autant à des dianhydrohexitols sous forme liquide que solide.

Ceci étant dit, le document WO 2012 / 042187 indique que pour des dianhydrohexitols sous forme solides, quand bien même ceux-ci seraient purifiés, stabilisés et conditionnés selon les techniques précédemment décrites, il est encore nécessaire de les mettre sous forme de pastilles pour être tout à fait certain qu'on évite le phénomène de mottage, particulièrement gênant dans les opérations de transport, de manipulation et surtout de transvasement des produits.

Si on suit le cheminement des travaux de la société demanderesse tel que relatés ci-dessus, la solution qui prévaut aujourd'hui pour conditionner de manière stable dans le temps des dianhydrohexitols consiste donc à utiliser des matériaux imperméables aux gaz, sous pression réduite en oxygène, voire également sous pression d'azote limitée. On comprend aisément qu'une telle démarche est à la fois longue et coûteuse. Par ailleurs, pour des dianhydrohexitols sous forme de poudre, il convient de prévoir en complément des opérations précitées une étape de pastillage en vue d'éviter des problèmes de mottage.

Or et contre toute attente, la société demanderesse a pu constater à l'issue de nombreux travaux qu'il existait un moyen très simple de conditionner de manière extrêmement stable dans le temps des dianhydrohexitols. Ce moyen repose sur le conditionnement dudit dianhydrohexitol sous forme de solution aqueuse dans un matériau d'emballage comprenant au moins une couche à base de polyoléfine telle que notamment le polyéthylène. De manière surprenante, la demanderesse a constaté que la stabilité d'un dianhydrohexitol conditionné sous forme liquide de cette manière est bien meilleure que celle d'un dianhydrohexitol solide emballé selon l'art antérieur cité ci-dessus.

Ceci est d'autant plus surprenant que ce type de matériau n'avait pas donné de bons résultats dans le cas de l'isosorbide solide, comme indiqué dans les exemples comparatifs du document WO 2009 / 019371 déjà cité. Par ailleurs, ceci va aussi à l'encontre de l'enseignement selon lequel il faudrait avoir recours à des matériaux de conditionnement imperméables aux gaz, sous pression réduite en oxygène, voire également sous pression d'azote limitée.

De plus et de manière tout à fait avantageuse, en limitant la quantité d'agents antioxydants dans le matériau d'emballage, on s'autorise des applications alimentaires et pharmaceutiques, chose qui n'était pas certaine dans le cas du document WO 2009 / 019371 qui recommande de travailler avec au moins 0,1 % en poids de ces additifs dans la couche barrière d'emballage.

On dispose donc d'un procédé de conditionnement particulièrement simple à mettre en œuvre, très peu coûteux et parfaitement adapté à des solutions aqueuses de dianhydrohexitol. Ce procédé permet notamment d'emballer et de conditionner de manière extrêmement stable dans le temps des solutions aqueuses de dianhydrohexitol. Dans la présente demande, cette stabilité est appréhendée de manière très simple à travers le suivi de l'évolution du pH de ladite composition au fil du temps. Une évolution ou dérive significative de ce pH est la preuve du phénomène d'oxydation du dianhydrohexitol, qui va conduire à la formation d'acide formique. Enfin, on dispose avantageusement de dianhydrohexitols qui peuvent être emballés, manipulés et transportés, dans le cadre d'applications finales dans les secteurs alimentaires et pharmaceutiques.

Le procédé objet de ladite demande concerne donc procédé de conditionnement d'un dianhydrohexitol, comprenant :
- la fourniture d'une solution aqueuse de dianhydrohexitol,
- l'introduction de ladite solution aqueuse de dianhydrohexitol dans un récipient,
- puis la fermeture dudit récipient,
dans lequel ledit récipient est constitué d'une seule couche, ladite couche étant à base de polyoléfine et ladite couche ne contenant pas plus de 0,1 % en poids d'antioxydant par rapport à son poids total et dans lequel la solution aqueuse de dianhydrohexitol présente une teneur en matière sèche entre 40 % et 95 % par rapport à son poids total.

L'expression « solution aqueuse de dianhydrohexitol » désigne une composition contenant essentiellement de l'eau et au moins un dianhydrohexitol. Elle est notamment caractérisée par sa matière sèche, exprimée en % en poids sec par rapport à son poids total. Celle-ci est comprise entre 40 % et 95 %, préférentiellement entre 50 % et 90 %, très préférentiellement entre 60 % et 85 %. Il convient par ailleurs de ne pas assimiler ladite solution aqueuse de dianhydrohexitol à une autre forme liquide de dianhydrohexitol : à savoir un fondu de dianhydrohexitol, c'est-à-dire un dianhydrohexitol dans un état fondu à une température supérieure ou égale à 63 +- 2°C (à pression atmosphérique).

Généralement, les dianhydrohexitols sont synthétisés en présence d'eau (ou de l'eau est générée au cours de leur synthèse) : en récupérant ledit dianhydrohexitol dans ce milieu réactionnel, on dispose immédiatement d'une solution aqueuse de dianhydrohexitol utilisable selon l'invention. Les solutions de dianhydrohexitols peuvent notamment être obtenues selon les procédés décrits dans les demandes de brevet précitées EP 1 287 000 et WO 03 / 043959. On peut choisir de conserver tout ou partie de l'eau utilisée lors de la préparation du dianhydrohexitol ou d'éliminer en totalité l'eau pour obtenir un produit sous forme solide qu'on remettra en solution aqueuse par simple ajout d'eau, ce qui constitue une autre possibilité pour préparer une solution aqueuse de dianhydrohexitol utilisable selon l'invention.

La solution aqueuse en question peut contenir un dianhydrohexitol seul, comme elle peut en contenir plusieurs. Ces dianhydrohexitols (1,4 - 3, 6-dianhydrohexitols) englobent l'isosorbide (1,4 - 3, 6-dianhydrosorbitol), l'isomannide (1,4 - 3,6 dianhydromannitol), l'isoidide (1,4 - 3, 6-dianhydroiditol) et les mélanges d'au moins deux de ces produits. De préférence, la solution aqueuse ne contient qu'un seul dianhydrohexitol qui est l'isosorbide.

Par « procédé de conditionnement », on entend une opération qui consiste à placer un produit dans un contenant, avec lequel il est en contact direct, afin de faciliter sa protection et sa conservation.

Le terme « couche » désigne dans la présente demande une enveloppe uniforme ; cette couche étant « à base » de polyoléfine, ce qui signifie qu'elle est constituée majoritairement de ladite polyoléfine (ladite polyoléfine représentant alors au moins 90 % en poids, préférentiellement 99 %, très préférentiellement 100 % en poids du poids total de ladite « couche »).

La polyoléfine peut être choisie en particulier parmi le polyéthylène, le polypropylène et les copolymères d'éthylène ou de propylène. De manière préférée, il s'agit de polyéthylène, plus préférentiellement de polyéthylène à haute densité (PEHD).

Par PEHD, on entend un polyéthylène de masse moléculaire en poids supérieure à 20 000 g /mol et présentant une zone de fusion entre 90 et 160°C. Ce polymère peut-être de couleur naturelle blanche ou teinté à l'aide d'un colorant, par exemple bleu, à condition que le colorant soit agréé au contact alimentaire. Il peut être aussi de couleur noire par addition de noir de carbone, sous réserve qu'il n'en contienne pas plus de 0.1 % en poids par rapport à son poids total.

Le récipient est constitué d'une seule couche, c'est-à-dire qu'il est constitué d'un matériau monocouche, qui est donc à base de polyoléfine.

La couche à base de polyoléfine ne contient pas plus de 0,1 % en poids d'antioxydant par rapport à son poids total. Préférentiellement, elle n'en contient pas plus de 0,01 % en poids par rapport à son poids total, et de manière la plus préférée, elle ne contient pas d'antioxydant.

Le terme agent « anti-oxydant » revêt dans la présente demande exactement le même sens que dans le document WO 2009 / 019371, à savoir qu'il englobe tous les composés capables de limiter ou de supprimer la dégradation thermo-oxydative, également connue sous le terme d'auto-oxydation, de composés organiques, en particulier de polymères organiques.

Une liste non exhaustive de ces composés est donnée dans le chapitre 1, intitulé « Antioxidants », de la 5ème édition de l'ouvrage « Plastics Additives Handbook » (2001, Cari Hanser Verlag, Munich (Allemagne). Lorsqu'un antioxydant est éventuellement présent dans la couche de polyoléfine selon la présente invention, il sera préférentiellement choisi parmi les donneurs d'hydrogène tels que les aminés aromatiques secondaires et les phénols à encombrement stérique important, les agents de décomposition des hydroperoxydes à base de phosphore tels que les phosphites et phosphonites, et ceux à base de soufre tels que les esters d'acide 3, 3-thiodipropionique, et les agents piégeurs de radicaux libres tels que le noir de carbone, les aminés à fort encombrement stérique, les hydroxylamines, les dérivés de benzofuranone et les phénols modifiés par des groupes acryloyle.

L'épaisseur globale du récipient ne joue pas un rôle déterminant dans la présente invention. Elle peut notamment être comprise entre 50 µm et 1 cm. Le récipient est de préférence un matériau rigide de manière à pouvoir supporter les contraintes liées à l'emballage d'un produit sous forme liquide comme la solution aqueuse selon la présente invention. Il peut s'agir d'un fût, d'un container, d'un réservoir, mais plus généralement, il s'agit donc d'un récipient rigide, de toute forme et de toute taille que ce soit. Il peut aussi s'agir d'un emballage souple, de préférence maintenu dans une structure rigide permettant de supporter les contraintes liées à l'emballage d'un produit sous forme liquide, par exemple un « Bag-in-box » ou un « Flexi-tank ».

Le procédé selon la présente invention comprend donc l'introduction dans le récipient précédemment décrit, la solution aqueuse de dianhydrohexitol précédemment décrite. Cette introduction est réalisée par tous les moyens bien connus de l'homme du métier, notamment des moyens adaptés à la manipulation et au transvasement de produits sous forme liquide, notamment de composition de dianhydrohexitol sous forme liquide. Ces moyens sont, par exemple le remplissage par voie gravitaire à partir d'une cuve de stockage située au-dessus du matériau d'emballage par l'intermédiaire d'un orifice de remplissage, ou à l'aide d'un équipement de pompage pour transvaser la solution aqueuse de dianhydrohexitol de la cuve au matériau d'emballage.

On procède ensuite à la fermeture du récipient. Cette fermeture a lieu grâce à des dispositifs connus de l'homme du métier, et adaptés à la forme du matériau en question. Ces dispositifs sont par exemple la fermeture de l'orifice de remplissage par l'intermédiaire d'un bouchon vissé ou serti à l'aide d'une sertisseuse.

Un autre objet de la présente invention concerne une solution aqueuse de dianhydrohexitol conditionnée dans un récipient, ledit récipient étant constitué d'une seule couche, ladite couche étant à base de polyoléfine et ladite couche ne contenant pas plus de 0,1 % en poids d'antioxydant par rapport à son poids total, et dans lequel ladite solution aqueuse de dianhydrohexitol présente une teneur en matière sèche comprise entre 40 % et 95 % par rapport à son poids total.

En d'autres termes, l'invention concerne un récipient qui contient ou dans lequel est placée une solution aqueuse de dianhydrohexitol, ledit récipient étant constitué d'une seule couche, ladite couche étant à base de polyoléfine et ladite couche ne contenant pas plus de 0,1 % en poids d'antioxydant par rapport à son poids total, et dans lequel ladite solution aqueuse de dianhydrohexitol présente une teneur en matière sèche comprise entre 40 % et 95 % par rapport à son poids total.

Dans ce cadre, la solution aqueuse de dianhydrohexitol d'une part, et la couche à base de polyoléfine d'autre part reprennent toutes les caractéristiques précédemment énumérées au sujet du procédé qui constitue le premier objet de la présente demande.

La teneur en matière sèche de la solution aqueuse de dianhydrohexitol est comprise entre 40 % et 95 %, préférentiellement entre 50 % et 90 %, très préférentiellement entre 60 % et 85 % par rapport à son poids total. Comme expliqué précédemment, cette solution aqueuse ne recouvre pas ce qu'on appelle un fondu.

La solution aqueuse peut contenir un dianhydrohexitol seul, comme elle peut en contenir plusieurs. Ces dianhydrohexitols (1,4 - 3, 6-dianhydrohexitols) englobent l'isosorbide (1,4 - 3, 6-dianhydrosorbitol), l'isomannide (1,4 - 3,6 dianhydromannitol), l'isoidide (1,4 - 3, 6-dianhydroiditol) et les mélanges d'au moins deux de ces produits. De préférence, la solution aqueuse ne contient qu'un seul dianhydrohexitol qui est l'isosorbide.

S'agissant du récipient, la polyoléfine peut être choisie en particulier parmi le polyéthylène, le polypropylène ou les copolymères d'éthylène et de propylène. De manière préférée, il s'agit de polyéthylène, plus préférentiellement de polyéthylène à haute densité (PEHD).

Par PEHD, on entend un polyéthylène de masse moléculaire en poids supérieure à 20 000 g /mol et présentant une zone de fusion entre 90 et 160°C. Ce polymère peut-être de couleur naturelle blanche ou teinté à l'aide d'un colorant, par exemple bleu, à condition que le colorant soit agréé au contact alimentaire. Il peut être aussi de couleur noire par addition de noir de carbone, sous réserve qu'il n'en contienne pas plus de 0.1 % en poids par rapport à son poids total.

Le récipient est constitué d'une seule couche, c'est-à-dire qu'il est constitué d'un matériau monocouche qui est donc à base de polyoléfine.

La couche à base de polyoléfine ne contient pas plus de 0,1 % en poids d'antioxydant par rapport à son poids total. Préférentiellement, elle n'en contient pas plus de 0,01 % en poids par rapport à son poids total, et de manière la plus préférée, elle ne contient pas d'antioxydant.

L'épaisseur globale du matériau d'emballage ne joue pas un rôle déterminant dans la présente invention. Elle peut notamment être comprise entre 50 µm et 1 cm. Le matériau d'emballage est de préférence un matériau rigide de manière à pouvoir supporter les contraintes liées à l'emballage d'un produit sous forme liquide comme la solution aqueuse selon la présente invention. Il peut s'agir d'un fût, d'un container, d'un réservoir, mais plus généralement, il s'agit donc d'un récipient rigide, de toute forme et de toute taille que ce soit. Il peut aussi s'agir d'un emballage souple, de préférence maintenu dans une structure rigide permettant de supporter les contraintes liées à l'emballage d'un produit sous forme liquide, par exemple un « Bag-in-box » ou un « Flexi-tank ».

Un dernier objet divulgué dans le présent document concerne l'utilisation de la solution aqueuse de dianhydrohexitol précitée, dans la fabrication de plastique, comme intermédiaire de synthèse chimique, dans les industries alimentaires et pharmaceutiques, et plus préférentiellement dans les industries alimentaires et pharmaceutiques.

L'invention pourra être encore mieux appréhendée à la lueur des exemples qui suivent mais qui ne sauraient en aucun cas être limitatifs.

### EXEMPLES

Les exemples suivants illustrent l'intérêt du procédé d'emballage selon l'invention, en démontrant qu'on parvient alors à emballer une composition de dianhydrohexitol (en l'occurrence d'isosorbide) qui reste très stable dans le temps, comme en témoigne l'évolution de son pH.

Non seulement le pH de la composition liquide d'isosorbide reste stable sur plusieurs mois quelles que soient les conditions de stockage, mais il est encore plus stable que le pH de la même composition d'isosorbide sous forme solide.

### Exemple 1

Cet exemple porte sur l'emballage et le stockage d'isosorbide, sous forme solide ou liquide, ledit isosorbide ayant au préalable été stabilisé avec du phosphate disodique.

On commence tout d'abord par fabriquer une composition d'isosorbide solide et une composition d'isosorbide liquide de la manière suivante :
Dans un réacteur agité double enveloppe, on introduit 1 kg d'une solution de sorbitol à 70 % de matière sèche commercialisée par la société demanderesse sous l'appellation NEOSORB 70/02 et 7 g d'acide sulfurique concentré. Le mélange obtenu est chauffé sous vide (pression d'environ 100 mbars) pendant 5 heures de façon à éliminer l'eau contenue dans le milieu réactionnnel initial et celle provenant de la réaction de déshydratation du sorbitol.

Le brut réactionnel est ensuite refroidi vers 100 °C puis neutralisé avec 11,4 g d'une solution de soude à 50 % (en poids). La composition d'isosorbide ainsi neutralisée est ensuite distillée sous vide (pression inférieure à 50 mbars).

Le distillat brut d'isosorbide, légèrement coloré (couleur jaune claire) est ensuite mis en solution dans le 2-propanol, à une température de 60 °C, de façon à obtenir une solution à 75 % de MS. Cette solution est ensuite refroidie lentement, en l'espace de 5 heures, jusqu' à une température de 10 °C. une amorce d'isosorbide recristallisé est ajoutée à 40 °C.

Les cristaux sont ensuite essorés dans une essoreuse et lavés avec du 2-propanol. Après séchage sous vide, les cristaux sont remis en solution dans l'eau de manière à obtenir une MS de 40 %.

Cette solution est ensuite percolée sur une colonne de charbon actif granulaire CPG 12-40 à une vitesse de 0,5 BV/h (Bed Volume ou Volume de lit / heure). La composition d'isosorbide décolorée ainsi obtenue est ensuite passée, à une vitesse de 2 BV/h successivement sur une colonne de résine cationique forte PUROLITE C 150 S puis une colonne de résine anionique forte AMBERLITE IRA 910. Cette solution est ensuite traitée par du charbon actif en poudre de type NORIT SX+ à 20°C pendant 1 heure. Le charbon actif est mis en œuvre à raison de 0,5 % en poids sec / poids sec de solution.

On introduit alors dans ladite composition 0,005 % de phosphate disodique (poids sec / poids sec d'isosorbide contenu dans la composition).

Après filtration, la solution d'isosorbide est concentrée sous vide. La solution est concentrée jusqu'à l'obtention d'une solution à 80% de matière sèche. Une partie de la solution est récupérée pour la réalisation des essais 4 et 5. On poursuit ensuite la concentration sous vide du reste de la solution pour l'élimination de l'eau résiduelle. La masse fondue obtenue cristallise au refroidissement sous forme d'un massé de gros cristaux que l'on broye ensuite pour obtenir une poudre de couleur blanche présentant une humidité de 0,2 %. Cette poudre est récupérée pour les essais 1, 2 et 3.

Ces différentes compositions servent ensuite à illustrer le procédé d'emballage selon l'invention ou selon l'art antérieur.

### Essai n° 1

Cet essai est relatif à l'art antérieur et illustre l'emballage de la composition d'isosorbide solide dans un conditionnement « PE + Alu ». Plus précisément, ce conditionnement consiste en une première sache interne (20 cm x 20 cm) en polyéthylène (PE) de 100 µm d'épaisseur associée à une seconde sache externe (25 cm x 25 cm) consistant en un complexe aluminium (Alu) contenant 80 µm d'épaisseur de polyéthylène recouvert de 8,5 µm d'épaisseur d'aluminium.

La composition d'isosorbide est emballée de la manière suivante : 100 g de la composition d'isosorbide solide comme obtenus précédemment sont introduits dans la sache interne PE qui est fermée par soudage à l'aide d'une soudeuse thermique à impulsions (modèle SZ 380 commercialisé par la société Joisten & Kettenbaum GmbH & Co, Bergisch Gladbach, Allemagne). Cette sache est elle-même placée à l'intérieur de la sache externe Alu, qui est ensuite fermée par soudage avec la même thermosoudeuse afin d'assurer l'étanchéité vis-à-vis de l'atmosphère extérieure.

### Essai n° 2

Cet essai est relatif à l'art antérieur et illustre l'emballage de la composition d'isosorbide solide dans un conditionnement consistant en « Liner BigBag PE blanc/alu/PET ». Plus précisément, ce conditionnement consiste en une sache (25 cm x 25 cm) constituée d'un complexe d'épaisseur totale d'environ 100 µm constitué d'une couche interne en polyéthylène (PE) d'épaisseur 80 µm, d'une couche intermédiaire en aluminium d'épaisseur 9µm et d'une couche externe en polyéthylène téréphtalate (PET) d'épaisseur 12 µm.

La composition d'isosorbide est emballée de la manière suivante : 100 g de la composition d'isosorbide solide comme obtenus précédemment sont introduits dans la sache « Liner BigBag PE blanc/alu/PET » qui est fermée par soudage à l'aide d'une soudeuse thermique à impulsions (modèle SZ 380 commercialisé par la société Joisten & Kettenbaum GmbH & Co, Bergisch Gladbach, Allemagne afin d'assurer l'étanchéité vis-à-vis de l'atmosphère extérieure.

### Essai n° 3

Cet essai est relatif à l'art antérieur et illustre l'emballage de la composition d'isosorbide solide dans un conditionnement consistant en « PE carbone + Alu ». Plus précisément, ce conditionnement consiste en une première sache interne (20 cm x 20 cm) en polyéthylène additivé carbone (PE carbone) de 150 µm d'épaisseur et d'une seconde sache (25 cm x 25 cm), externe, consistant en un complexe aluminium (Alu) contenant 80 µm d'épaisseur de polyéthylène recouverts de 8,5 µm d'épaisseur d'aluminium.

La composition d'isosorbide est emballée de la manière suivante : 100 g de la composition d'isosorbide solide comme obtenus précédemment sont introduits dans la sache interne PE carbone qui est fermée par soudage à l'aide d'une soudeuse thermique à impulsions (modèle SZ 380 commercialisé par la société Joisten & Kettenbaum GmbH & Co, Bergisch Gladbach, Allemagne). Cette sache est elle-même placée à l'intérieur de la sache externe Alu, qui est ensuite fermée par soudage avec la même thermosoudeuse afin d'assurer l'étanchéité vis-à-vis de l'atmosphère extérieure.

### Essai n° 4

Cet essai est relatif à l'invention et illustre l'emballage de la composition d'isosorbide liquide dans un conditionnement en « PEHD blanc ». Plus précisément, ce conditionnement consiste en un flacon de 150 ml en PEHD de couleur naturelle ne contenant pas d'antioxydants ni de colorants.

La composition d'isosorbide est emballée de la manière suivante : 100 g de la composition d'isosorbide liquide comme obtenus précédemment sont versés à l'intérieur du flacon PEHD blanc qui est fermé par vissage d'un bouchon constitué du même matériau afin d'assurer l'étanchéité vis-à-vis de l'atmosphère extérieure.

### Essai n° 5

Cet essai est relatif à l'invention et illustre l'emballage de la composition d'isosorbide liquide dans un conditionnement en « PEHD noir ». Plus précisément, ce conditionnement consiste en un flacon de 150 ml en PEHD de couleur noire contenant moins de 0.1% de colorant noir.

La composition d'isosorbide est emballée de la manière suivante : 100 g de la composition d'isosorbide liquide comme obtenus précédemment sont versés à l'intérieur du flacon PEHD noir qui est fermé par vissage d'un bouchon constitué du même matériau afin d'assurer l'étanchéité vis-à-vis de l'atmosphère extérieure.

Les compositions d'isosorbide solides et liquides emballées selon les essais 1 à 5 sont placées dans une étuve ventilée, thermostatée à la température de 50 °C. Plusieurs conditionnements par essai sont placés dans l'étuve afin de suivre l'évolution du pH de chaque composition au cours du temps.

On suit l'évolution du pH de chaque composition au cours du temps de la manière suivante : dans un premier temps, pour chaque composition d'isosorbide solide ou liquide, la totalité de l'échantillon est extraite des matériaux de conditionnement et est additionnée à de l'eau osmosée de manière à obtenir une solution d'isosorbide à 40 % de matière sèche dans de l'eau osmosée, puis on mesure le pH initial de cette solution. La mesure de pH est effectuée sur un pHmètre de marque RADIOMETER ANALYTICAL PHM 220 équipé d'une électrode combinée à fil Ag/AgCI de marque METTLER TOLEDO, préalablement étalonnée à l'aide de solutions tampon pH 7 et 4. Après une période déterminée de stockage à 50°C, on prépare de la même manière une solution d'isosorbide à 40 % de matière sèche dans de l'eau osmosée pour chaque composition d'isosorbide solide ou liquide, puis on effectue la mesure de pH à l'aide du même pH mètre.

Les résultats sont reportés dans le tableau 1. Ils démontrent clairement que la solution aqueuse d'isosorbide conditionnée selon l'invention dans un emballage « PEHD noir » et « PEHD blanc » est bien plus stable que l'isosorbide solide conditionné dans les emballages « PE + Alu », « Liner BigBag PE blanc/alu/PET » et « PE carbone + Alu ».

**Tableau 1**

| Essai n° | 1 | 2 | 3 | 4 | 5 |
|---|---|---|---|---|---|
| Emballage | PE Blanc + Alu | Liner BigBag PE blanc/alu/PET | PE Carbone + Alu | HDPE blanc | HDPE noir |
| Forme physique | Solide | Solide | Solide | Liquide | Liquide |
| pH | | | | | |
| 0 jour | 7,2 | 7,6 | 7,2 | 7,4 | 7,4 |
| 1 mois | 3,1 | 3,9 | 3,2 | 7,2 | 7,2 |
| 2 mois | | | | 7,1 | 7,2 |
| 3 mois | | | | 4,0 | 6,8 |

### Exemple 2

Cet exemple porte sur l'emballage et le stockage d'isosorbide, sous forme solide ou liquide, ledit isosorbide ayant au préalable été stabilisé avec de la diéthanolamine.

On commence tout d'abord par fabriquer une composition d'isosorbide liquide et une composition d'isosorbide solide de la même manière que pour l'exemple 1 à l'exception du fait qu'on remplace le phosphate disodique par 0.0025 % de diéthanolamine en poids sec par rapport au poids sec d'isosorbide.

Les compositions liquides et solides obtenues comme précédemment servent ensuite à illustrer le procédé d'emballage selon l'invention ou selon l'art antérieur.

### Essai n° 6

Cet essai est relatif à l'art antérieur et illustre l'emballage de la composition d'isosorbide solide dans un conditionnement « PE + Alu ». Plus précisément, ce conditionnement consiste en une première sache interne (20 cm x 20 cm) en polyéthylène (PE) de 100 µm d'épaisseur associée à une seconde sache externe (25 cm x 25 cm) consistant en un complexe aluminium (Alu) contenant 80 µm d'épaisseur de polyéthylène recouvert de 8,5 µm d'épaisseur d'aluminium.

La composition d'isosorbide est emballée de la manière suivante : 100 g de la composition d'isosorbide solide comme obtenus précédemment sont introduits dans la sache interne PE qui est fermée par soudage à l'aide d'une soudeuse thermique à impulsions (modèle SZ 380 commercialisé par la société Joisten & Kettenbaum GmbH & Co, Bergisch Gladbach, Allemagne). Cette sache est elle-même placée à l'intérieur de la sache externe Alu, qui est ensuite fermée par soudage avec la même thermosoudeuse afin d'assurer l'étanchéité vis-à-vis de l'atmosphère extérieure.

### Essai n° 7

Cet essai est relatif à l'art antérieur et illustre l'emballage de la composition d'isosorbide solide dans un conditionnement consistant en « Liner BigBag PE blanc/alu/PET ». Plus précisément, ce conditionnement consiste en une sache (25 cm x 25 cm) constituée d'un complexe d'épaisseur totale d'environ 100 µm constitué d'une couche interne en polyéthylène (PE) d'épaisseur 80µm, d'une couche intermédiaire en aluminium d'épaisseur 9µm et d'une couche externe en polyéthylène téréphtalate (PET) d'épaisseur 12µm.

La composition d'isosorbide est emballée de la manière suivante : 100 g de la composition d'isosorbide solide comme obtenus précédemment sont introduits dans la sache « Liner BigBag PE blanc/alu/PET » qui est fermée par soudage à l'aide d'une soudeuse thermique à impulsions (modèle SZ 380 commercialisé par la société Joisten & Kettenbaum GmbH & Co, Bergisch Gladbach, Allemagne afin d'assurer l'étanchéité vis-à-vis de l'atmosphère extérieure.

### Essai n° 8

Cet essai est relatif à l'invention et illustre l'emballage de la composition d'isosorbide liquide dans un conditionnement en « PEHD blanc ». Plus précisément, ce conditionnement consiste en un flacon de 150 ml en PEHD de couleur naturelle ne contenant pas d'antioxydants ni de colorants.

La composition d'isosorbide est emballée de la manière suivante : 100 g de la composition d'isosorbide liquide comme obtenus précédemment sont versés à l'intérieur du flacon PEHD blanc qui est fermé par vissage d'un bouchon constitué du même matériau afin d'assurer l'étanchéité vis-à-vis de l'atmosphère extérieure.

### Essai n° 9

Cet essai est relatif à l'invention et illustre l'emballage de la composition d'isosorbide liquide dans un conditionnement en « PEHD noir ». Plus précisément, ce conditionnement consiste en un flacon de 150 ml en PEHD de couleur noire contenant moins de 0.1% de colorant noir.

La composition d'isosorbide est emballée de la manière suivante : 100g de la composition d'isosorbide liquide comme obtenus précédemment sont versés à l'intérieur du flacon PEHD noir qui est fermé par vissage d'un bouchon constitué du même matériau afin d'assurer l'étanchéité vis-à-vis de l'atmosphère extérieure.

Les compositions d'isosorbide solides et liquides emballées selon les essais 6 à 9 sont placées dans une étuve ventilée, thermostatée à la température de 50°C. Plusieurs conditionnements par essai sont placés dans l'étuve afin de suivre l'évolution du pH de chaque composition au cours du temps.

On suit l'évolution du pH de chaque composition au cours du temps selon le même protocole que pour l'exemple 1
Les résultats figurent dans le tableau 2. Comme pour l'exemple 1, on constate que les solutions aqueuses d'isosorbide emballées selon l'invention, sont bien plus stables que les compositions solides d'isosorbide emballées selon l'art antérieur.

**Tableau 2**

| Essai n° | 6 | 7 | 8 | 9 |
|---|---|---|---|---|
| Emballage | PE Blanc + Alu | Liner BigBag PE blanc/alu/PET | HDPE blanc | HDPE noir |
| Forme physique | Solide | Solide | Liquide | Liquide |
| pH 0 jour | 8,1 | 8,4 | 8,2 | 8,2 |
| 1 mois | 6,5 | 7,5 | 7,7 | 8,2 |
| 2 mois | 3,2 | 7,5 | 7,6 | 8,2 |
| 3 mois | | 3,2 | 7,2 | 7,4 |

## Revendications

1. - Procédé de conditionnement d'un dianhydrohexitol, comprenant :
- la fourniture d'une solution aqueuse de dianhydrohexitol,
- l'introduction de ladite solution aqueuse de dianhydrohexitol dans un récipient,
- puis la fermeture dudit récipient,
**caractérisé en ce que** ledit récipient est constitué d'une seule couche, ladite couche étant à base de polyoléfine et ladite couche ne contenant pas plus de 0,1 % en poids d'antioxydant par rapport à son poids total et dans lequel la solution aqueuse de dianhydrohexitol présente une teneur en matière sèche entre 40 % et 95 % par rapport à son poids total.

2. - Procédé selon la revendication 1, **caractérisé en ce que** la polyoléfine est choisie parmi le polyéthylène, le polypropylène ou les copolymères d'éthylène et de propylène, est préférentiellement le polyéthylène, plus préférentiellement le polyéthylène à haute densité (PEHD).

3. - Procédé selon l'une quelconque des revendications 1 à 2, **caractérisé en ce que** le dianhydrohexitol est choisi parmi l'isosorbide, l'isomannide, l'isoidide ou les mélanges d'au moins deux de ces produits, est préférentiellement l'isosorbide.

4. - Procédé selon l'une quelconque des revendications 1 à 3, **caractérisé en ce que** la solution aqueuse de dianhydrohexitol présente une teneur en matière sèche entre 50 % et 90 %, préférentiellement entre 60 % et 85 % par rapport à son poids total.

5. - Récipient contenant une solution aqueuse de dianhydrohexitol, **caractérisé en ce que** ledit récipient est constitué d'une seule couche, ladite couche étant à base de polyoléfine et ladite couche ne contenant pas plus de 0,1 % en poids d'antioxydant par rapport à son poids total, et dans lequel ladite solution aqueuse de dianhydrohexitol présente une teneur en matière sèche comprise entre 40 % et 95 % par rapport à son poids total.

6. - Récipient contenant une solution aqueuse de dianhydrohexitol selon la revendication 5, **caractérisé en ce que** la polyoléfine est choisie parmi le polyéthylène, le polypropylène ou les copolymères d'éthylène et de propylène, et est préférentiellement le polyéthylène, plus préférentiellement le polyéthylène à haute densité (PEHD).

7. - Récipient contenant une solution aqueuse de dianhydrohexitol selon l'une quelconque des revendications 5 à 6, **caractérisé en ce que** le dianhydrohexitol est choisi parmi l'isosorbide, l'isomannide, l'isoidide ou les mélanges d'au moins deux de ces produits, est préférentiellement l'isosorbide.

8. - Récipient contenant une solution aqueuse de dianhydrohexitol selon l'une quelconque des revendications 5 à 7, **caractérisé en ce que** ladite solution de dianhydrohexitol présente une teneur en matière sèche comprise entre 50 % et 90 %, préférentiellement entre 60 % et 85 % par rapport à son poids total.

## Patentansprüche

1. Verfahren zur Konditionierung eines Dianhydrohexitols, umfassend:
- Bereitstellen einer wässrigen Dianhydrohexitol-Lösung,
- Einbringen der wässrigen Dianhydrohexitol-Lösung in einen Behälter,
- anschließendes Verschließen des Behälters,
**dadurch gekennzeichnet, dass** der Behälter aus einer einzigen Schicht besteht, wobei die Schicht auf Polyolefin basiert und die Schicht nicht mehr als 0,1 Gew.-% Antioxidationsmittel, bezogen auf ihr Gesamtgewicht, enthält und wobei die wässrige Dianhydrohexitol-Lösung einen Trockensubstanzgehalt zwischen 40 % und 95 %, bezogen auf ihr Gesamtgewicht, aufweist.

2. Verfahren nach Anspruch 1, **dadurch gekennzeichnet, dass** das Polyolefin aus Polyethylen, Polypropylen oder Copolymeren von Ethylen und Propylen gewählt ist, bevorzugt Polyethylen, stärker bevorzugt Polyethylen hoher Dichte (PEHD).

3. Verfahren nach einem der Ansprüche 1 bis 2, **dadurch gekennzeichnet, dass** das Dianhydrohexitol gewählt ist aus Isosorbid, Isomannid, Isoidid oder Mischungen aus wenigstens zwei dieser Produkte, und bevorzugt Isosorbid ist.

4. Verfahren nach einem der Ansprüche 1 bis 3, **dadurch gekennzeichnet, dass** die wässrige Dianhydrohexitol-Lösung einen Trockengehalt zwischen 50 % und 90 %, bevorzugt zwischen 60 % und 85 % ihres Gesamtgewichts aufweist.

5. Behälter, der eine wässrige Dianhydrohexitol-Lösung enthält, **dadurch gekennzeichnet, dass** der Behälter aus einer einzigen Schicht besteht, wobei die Schicht auf Polyolefin basiert und nicht mehr als 0,1 Gew.-% Antioxidationsmittel, bezogen auf ihr Gesamtgewicht, enthält, und wobei die wässrige Dianhydrohexitol-Lösung einen Trockensubstanzgehalt von 40 bis 95 % ihres Gesamtgewichts aufweist.

6. Behälter, der eine wässrige Dianhydrohexitol-Lösung nach Anspruch 5 enthält, **dadurch gekennzeichnet, dass** das Polyolefin aus Polyethylen, Polypropylen oder Copolymeren von Ethylen und Propylen gewählt ist und bevorzugt Polyethylen, stärker bevorzugt Polyethylen hoher Dichte (PEHD) ist.

7. Behälter, der eine wässrige Dianhydrohexitol-Lösung nach einem der Ansprüche 5 bis 6 enthält, **dadurch gekennzeichnet, dass** das Dianhydrohexitol aus Isosorbid, Isomannid, Isoidid oder Mischungen aus wenigstens zwei dieser Produkte gewählt ist, und bevorzugt Isosorbid ist.

8. Behälter, der eine wässrige Dianhydrohexitol-Lösung nach einem der Ansprüche 5 bis 7 enthält, **dadurch gekennzeichnet, dass** die Dianhydrohexitol-Lösung einen Trockengehalt von 50 % bis 90 %, bevorzugt von 60 % bis 85 %, bezogen auf ihr Gesamtgewicht, aufweist.

## Claims

1. A method for packaging a dianhydrohexitol, comprising:
- providing an aqueous dianhydrohexitol solution,
- introducing said aqueous dianhydrohexitol solution into a container,
- and then closing said container,
**characterised in that** said container is made of one single layer, said layer being polyolefin based and said layer containing no more than 0.1% by weight of antioxidant relative to its total weight and wherein the aqueous dianhydrohexitol solution has a dry solids content between 40% and 95% relative to its total weight.

2. The method according to claim 1, **characterised in that** the polyolefin is selected from among polyethylene, polypropylene, or copolymers of ethylene and propylene, is preferably polyethylene, more preferably high-density polyethylene (HDPE).

3. The method according to any one of claims 1 to 2, **characterised in that** the dianhydrohexitol is selected from among isosorbide, isomannide, isoidide or mixtures of at least two of these products, is preferably isosorbide.

4. The method according to any one of claims 1 to 3, **characterised in that** the aqueous dianhydrohexitol solution has a dry solids content between 50% and 90%, preferably between 60% and 85% relative to its total weight.

5. A container containing an aqueous dianhydrohexitol solution, **characterised in that** said container is made of one single layer, said layer being polyolefin based and said layer containing no more than 0.1% by weight of antioxidant relative to its total weight, and wherein said aqueous dianhydrohexitol solution has a dry solids content of between 40% and 95% relative to its total weight.

6. The container containing an aqueous dianhydrohexitol solution according to claim 5, **characterised in that** the polyolefin is selected from among polyethylene, polypropylene, or copolymers of ethylene and propylene, and is preferably polyethylene, more preferably high-density polyethylene (HDPE).

7. The container containing an aqueous dianhydrohexitol solution according to any one of claims 5 to 6, **characterised in that** the dianhydrohexitol is selected from among isosorbide, isomannide, isoidide or mixtures of at least two of these products, is preferably isosorbide.

8. The container containing an aqueous dianhydrohexitol solution according to any one of claims 5 to 7, **characterised in that** said dianhydrohexitol solution has a dry solids content of between 50% and 90%, preferably between 60% and 85% relative to its total weight.
